# EUROPEAN PATENT APPLICATION

(11) **EP 1 671 550 A1**
(43) Date of publication of application: **21.06.2006**
(21) Application number: 05006027.6
(22) Date of filing: 18.03.2005
(51) Int. Cl.: A23L 1/30, A61K 36/718, A23L 1/302, A23L 1/305, A23L 1/29, A23L 2/52

(54) **Energy drink compositions**

(30) Priority: 15.12.2004 US 14676
(71) Applicant: Herbalife International, Inc., Century City, California 90067 (US)
(72) Inventor: Krumhar, Kim C., Carlsbad, CA 92009 (US); Lam, May, Torrance, CA 90504 (US)
(74) Representative: Ebner von Eschenbach, Jennifer

(57) **Abstract**

A composition that may be mixed with a fluid comprises a vitamin B, vitamin C, taurine, an extract of ginkgo biloba, and an extract of guarana.

## Description

### TECHNICAL FIELD

This disclosure relates to compositions for beverages.

### BACKGROUND

With the ever-increasing pace of life in modern times, the general population is exposed to increasing mental and physical stress. A large number of products have been introduced that contain various ingredients aimed at helping counter one or more aspects of such stress. There is a very large number of ingredients available. Various combinations of such ingredients exhibit varying levels of efficacy based upon the relative concentrations as well as the interaction between the ingredients and the body's response to such combinations. Therefore, developing new combinations of ingredients is largely a matter of physical experimentation.

The embodiments of the present disclosure provide new formulations of ingredients for human consumption to assist the mental and/or physical processes of the consumer.

### SUMMARY

In a first embodiment disclosed herein, a composition that may be mixed with a fluid comprises a vitamin B, vitamin C, taurine, an extract of ginkgo biloba, and an extract of guarana.

In another embodiment disclosed herein, the composition may further comprise one or more of an extract of ginseng, caffeine, effervescents, sweeteners, salts, lubricants, preservatives, excipients, flavorants and colorants.

In a further embodiment disclosed herein, the composition may be dissolved in water for consumption.

### DETAILED DESCRIPTION

The present disclosure provides novel compositions for beverages that offer refreshment to the drinker combined with health benefits. In one exemplary embodiment disclosed herein, a beverage composition includes herbs, minerals, and vitamins that are believed to impart the drinker a boost in energy and an overall enhanced feeling of well-being.

The herbs in the composition may include any one or more of ginkgo biloba, guarana, and ginseng. Ginkgo biloba is known to provide nutritional support for mental alertness, enhanced vitality level, circulatory health and blood vessel health. It also has high antioxidant activity that is valuable for fighting age related conditions. Ginkgo biloba is known to increase blood flow to the brain and throughout the body's network of blood vessels that supply blood and oxygen to the organ systems. It also increases metabolism efficiency, regulates neurotransmitters, and boosts oxygen levels in the brain. Benefits of enhanced circulation in the brain include improved short and long term memory, increased reaction time, and improved mental clarity.

Guarana is a concentrated source of caffeine, and consumption is believed to offer health benefits including stimulating the heart and central nervous system, enhancing alertness, and alleviating fatigue. It also has strong diuretic activity and reduces constriction of the bronchials, aiding the consumer to breathe more freely.

Ginseng is commonly used as an adaptogen, i.e. it normalizes physical functioning depending on what the individual needs (for example, it will lower high blood pressure, but raise low blood pressure). It is also used to reduce the effects of stress, improve performance, boost energy levels, enhance memory, and stimulate the immune system. Ginseng helps maintain body functions, and has been shown to increase energy, stamina, and help the body resist viral infections and environmental toxins.

In a further exemplary embodiment, the composition may include vitamin C, a water-soluble vitamin whose health benefits are well documented as it is vital to the production of collagen by the body. Vitamin C is also important because it helps protect the fat-soluble vitamins A and E as well as fatty acids from oxidation.

In a further exemplary embodiment, the composition may include one or more B vitamins, including thiamine, riboflavin, niacin, pantothenic acid, pyrodixine, biotin, cyanocobalamin, choline and/or folic acid, including the reduced forms of folic acid such as (but not only) folinic acid, calcium folinate, and methyltetrahydrafolate. The B-complex vitamins are also water soluble vitamins that aid the breakdown of carbohydrates into glucose to provide energy for the body, the breakdown of fats and proteins to aid the normal functioning of the nervous system, and muscle tone in the stomach and intestinal tract. Particular forms of B vitamins in the composition may include d-Calcium pantothenate, niacinamide, pyridoxine hydrochloride, and thiamine mononitrate.

The composition may further include inositol, which is known to be necessary for the formation of lecithin and to functions closely with folacin, Vitamins B-6 and B-12, choline, betaine, and methionine to prevent the accumulation of fats in the liver.

Caffeine is known to be useful as a cardiac stimulant and also as a mild diuretic that increases urine production. Of course, caffeine is well known as a mental stimulant, due to its affinity for binding to the adenosine receptors in nerve cells. The caffeine in the composition may include anhydrous caffeine, and may also be provided by the guarana in the composition.

Taurine is an amino acid that functions in electrically active tissues such as the brain and heart to help stabilize cell membranes. It also has functions in the gallbladder, eyes, and blood vessels and is believed to possess antioxidant and detoxifying activity. Taurine aids the movement of potassium, sodium, calcium, and magnesium in and out of cells and thus helps generate nerve impulses. Taurine is also an inhibitory neurotransmitter, and it functions as a mild sedative in the brain.

In a further exemplary embodiment, the composition may contain an effervescent. As is understood, an effervescent is an agent comprising one or more compounds which, acting together or individually, evolve a gas on contact with water. The gas evolved is generally oxygen or, most commonly, carbon dioxide. Preferred effervescent agents comprise an acid component and a base component that react in the presence of water to generate carbon dioxide gas. The acid component can comprise one or more acids and the base component can comprise one or more bases. Preferably, the base component comprises an alkali metal or alkaline earth metal carbonate or bicarbonate and the acid component comprises an aliphatic carboxylic acid. Non-limiting examples of suitable bases for use in a base component include carbonate salts (e.g., calcium carbonate), bicarbonate salts (e.g., sodium bicarbonate), sesquicarbonate salts, and mixtures thereof. Sodium bicarbonate is a preferred base.

Non-limiting examples of suitable acids for use in an acid component include citric acid, lactic acid, glutaric acid, phosphoric acid, acetic acid, tartaric acid, malic acid, fumaric acid, adipic acid, succinic acid, oxaloacetate, acid anhydrides of such acids, acid salts of such acids, and mixtures thereof. Citric acid is a preferred acid.

In a further exemplary embodiment, the composition may contain sweeteners. Preferred sweeteners for use in the present invention are sugars and sugar alcohols such as sucrose, fructose, dextrose, maltose, lactose, high fructose corn syrup solids, invert sugar, sugar alcohols, including sorbitol, as well as mixtures of these sugars and sugar alcohols. In order to deliver lower levels of solids per dosage, it may be preferred to use a higher intensity sweetener with the sugar or sugar alcohol. These higher intensity sweeteners include saccharin, cyclamates, acesulfame K, L-aspartyl-L-phenylalanine lower alkyl ester sweeteners (e.g., aspartame); L-aspartyl-D-alanine amides; L-aspartyl-D-serine amides; L-aspartyl-L-1-hydroxymethylalkaneamide sweeteners; L-aspartyl-1-hydroxyethyalkaneamide sweeteners; and L-aspartyl-D-phenylglycine ester and amide sweeteners. Further sweeteners contemplated for use with the compositions disclosed herein include sweeteners derived from stevia, sweeteners derived from momordica grosvenorii, and sweeteners derived from mogrosides. A particularly preferred sweetener system is a combination of sucralose with acesulfame K and corn syrup solids.

In a further exemplary embodiment, the composition may include a salt. Non-limiting examples of salts may include preservatives such as sodium benzoate and potassium benzoate, and antacids such as potassium bicarbonate and sodium bicarbonate.

In a further exemplary embodiment, the composition may include a lubricant, such as Compitrol®. In one embodiment, the lubricant is composed at least partially of benzoates. In a further embodiment, the lubricant is composed at least partially of a polyethylene glycol with a molecular weight between 2,000 and 10,000.

In a further exemplary embodiment, the composition may include polyethylene glycol.

In another exemplary embodiment, the composition may be provided in tablet form, wherein the ingredients are compressed together with microcrystalline cellulose into a tablet. Microcrystalline cellulose offers unique compressibility and carrying capacity, and exhibits excellent properties as an excipient for solid dosage forms as it compacts well under minimum compression pressures, has high binding capability, and creates tablets that are extremely hard, stable, yet disintegrate rapidly. Other advantages include low friability, inherent lubricity, and high dilution potential. It must be understood that the composition of the present disclosure may include any other excipient or excipients, including but not limited to lactose, maltodextrins, granular fructose, granular sucrose, and any other crystalline sugary carbohydrates.

In another exemplary embodiment, the composition may be dissolved in water or other liquid suitable for human consumption.

The flavorants that may be included in the composition are not relevant to the inventive concepts disclosed herein, and those skilled in the art are familiar with the wide range of flavorants available. Therefore, any suitable flavorant or combination of flavorants, natural and/or artificial, are within the contemplated scope of the present disclosure.

In another exemplary embodiment, the composition may further include food colorants to improve the visual appearance of a drink prepared with the composition.

Table 1 lists one exemplary embodiment of ingredients and ranges of concentration for each exemplary ingredient in a composition in accordance with the present disclosure. The exemplary composition includes as one exemplary ingredient a composition named Blend #1, exemplary ingredients of which are listed in Table 2. Both tables list an upper and a lower exemplary limit for each particular ingredient in terms of weight percent of the composition. Those skilled in the art will appreciate and know how to select an actual weight percent for each ingredient chosen to be included in any particular embodiment of the compositions described herein.

**Table 1**

| Ingredient | Lower % | Upper % |
|---|---|---|
| Blend #1 | 50.0000% | 70.0000% |
| Sodium Bicarbonate | 10.0000% | 20.0000% |
| Corn Syrup Solids | 3.0000% | 9.0000% |
| Potassium Bicarbonate | 2.0000% | 8.0000% |
| Microcrystalline Cellulose | 2.0000% | 8.0000% |
| Panax Ginseng | 1.0000% | 5.0000% |
| Polyethylene Glycol | 1.0000% | 5.0000% |
| Ginkgo Biloba Extract | 0.5000% | 5.0000% |
| Sodium Benzoate | 0.5000% | 5.0000% |
| Acesulfame K | 0.2000% | 2.0000% |
| Sucralose Powder | 0.1000% | 5.0000% |

**Table 2**

| Ingredient | Lower % | Upper % |
|---|---|---|
| Anhydrous Citric Acid | 60.0000% | 95.0000% |
| Flavor | 1.2500% | 20.0000% |
| L-Taurine | 0.5000% | 12.0000% |
| Ascorbic Acid | 0.2500% | 8.0000% |
| Caffeine Powder, Anhydrous Natural | 0.2500% | 8.0000% |
| Guarana Extract | 0.0500% | 4.0000% |
| Biotin | 0.0500% | 4.0000% |
| Inositol | 0.0500% | 4.0000% |
| d-Calcium Pantothenate | 0.0500% | 4.0000% |
| Niacinamide | 0.0500% | 4. 0000% |
| Pyridoxine Hydrochloride USP | 0.0250% | 2.0000% |
| Thiamine Mononitrate Powder USP | 0.0050% | 2.0000% |
| Riboflavin USP | 0.0050% | 0.8000% |
| Cyanocobalamin | 0.0050% | 0.8000% |

Having now described the invention in accordance with the requirements of the patent statutes, those skilled in this art will understand how to make changes and modifications to the present invention to meet their specific requirements or conditions. Such changes and modifications may be made without departing from the scope of the invention as disclosed herein.

In broad summary, this writing has disclosed a composition that may be mixed with a fluid comprises a vitamin B, vitamin C, taurine, an extract of ginkgo biloba, and an extract of guarana. The composition may further comprise one or more of caffeine, an extract of ginseng, excipients, sweeteners, effervescents, salts, lubricants, preservatives, flavorants and colorants. The composition may be dissolved in water or other liquid for consumption.

## Claims

1. A composition that may be mixed with a fluid, comprising:
a vitamin B;
vitamin C;
taurine;
an extract of ginkgo biloba; and
an extract of guarana.

2. The composition of claim 1, further comprising:
an effervescent.

3. The composition of claim 2, wherein the effervescent comprises an acid component and a base component.

4. The composition of claim 3, wherein the base component comprises one or more selected from the group consisting of carbonate salts, bicarbonate salts, and sesquicarbonate salts.

5. The composition of claim 3 or 4, wherein the acid component comprises one or more selected from the group consisting of citric acid, lactic acid, glutaric acid, phosphoric acid, acetic acid, tartaric acid, malic acid, fumaric acid, adipic acid, succinic acid, oxaloacetate, an acid anhydride of any one of the foregoing acids, and an acid salt of any one of the foregoing acids.

6. The composition of claim 3 or 4, wherein the effervescent comprises:
citric acid; and
sodium bicarbonate.

7. The composition of any one of claims 1 - 6, further comprising:
a sweetener.

8. The composition of claim 7, wherein the sweetener comprises one or more selected from the group consisting of sucrose, fructose, dextrose, maltose, lactose, high fructose corn syrup solids, invert sugar, sugar alcohols, sorbitol, saccharin, cyclamates, sweeteners derived from stevia, sweeteners derived from momordica grosvenorii, sweeteners derived from mogrosides, acesulfame K, L-aspartyl-L-phenylalanine lower alkyl ester sweeteners, L-aspartyl-D-alanine amide sweeteners, L-aspartyl-D-serine amide sweeteners, L-aspartyl-L-1-hydroxymethylalkaneamide sweeteners, L-aspartyl-1-hydroxyethyalkaneamide sweeteners, and L-aspartyl-D-phenylglycine ester and amide sweeteners.

9. The composition of any one of claims 1-8, further comprising:
a flavorant.

10. The composition of any one of claims 1-9, further comprising:
a salt.

11. The composition of claim 10, wherein the salt comprises one or more selected from the group consisting of sodium benzoate, potassium benzoate, sodium bicarbonate, and potassium bicarbonate.

12. The composition of any one of claims 1-11, further comprising:
a lubricant.

13. The composition of claim 12, wherein the lubricant comprises one or more selected from the group consisting of benzoates, polyethylene glycols having a molecular weight between 2,000 and 10,000, and Compitrol®.

14. The composition of any one of claims 1-13, wherein the vitamin B comprises one or more selected from the group consisting of thiamine, riboflavin, niacin, pantothenic acid, pyrodixine, biotin, cyanocobalamin, folic acid, and reduced forms of folic acid.

15. The composition of claim 14, further comprising:
inositol.

16. The composition of claim 14, wherein the vitamin B comprises one or more selected from the group consisting of d-Calcium pantothenate, niacinamide, pyridoxine hydrochloride, and thiamine mononitrate.

17. The composition of any one of claims 1-16, further comprising:
an excipient.

18. The composition of claim 17, wherein the excipient comprises one or more selected from the group consisting of microcrystalline cellulose, lactose, maltodextrins, granular fructose, granular sucrose, and crystalline sugary carbohydrates.

19. The composition of any one of claims 1-18, further comprising:
an extract of ginseng.

20. The composition of any one of claims 1-19, further comprising:
caffeine.

21. The composition of claim 20, wherein the caffeine comprises anhydrous caffeine.

22. A beverage, comprising:
the composition of any one of claims 1-21 dissolved in water.
